# NEW EUROPEAN PATENT SPECIFICATION

(11) **EP 1 607 391 B2**
(45) Date of publication and mention of the opposition decision: **17.10.2012**
(45) Mention of the grant of the patent: 14.05.2008
(21) Application number: 05468012.9
(22) Date of filing: 10.06.2005
(51) Int. Cl.: C07D 251/64, C08G 12/32

(54) **Continuous process for production of methylol melamines and use thereof for production of highly etherified melamine resins**
Kontinuierliches Verfahren zur Herstellung von Methylolmelaminen und deren Verwendung zur Herstellung von hochveretherten Melaminharzen
Procédé continu pour la préparation de melamines de methylol pour la préparation de résines de melamine trés etherifiées

(30) Priority: 17.06.2004 SI 200400176
(43) Date of publication of application: 21.12.2005
(73) Proprietor: Melamin Kemicna tovarna d.d. Kocevje, 1330 Kocevje (SI)
(72) Inventor: Ogorelec, Primoz, 1339 Kocevje (SI); Aljaz-Rozic, Mateja, 1330 Kocevje (SI); Strnad, Tatjana, 1330 Kocevje (SI); Mihelic, Igor, 1331 Dolenja vas (SI); Skulj, Iztok, 1332 Stara Cerkev (SI)
(74) Representative: Viering, Jentschura & Partner

(56) References cited:
- EP-A- 0 355 760
- WO-A-01/60882

## Description

### Technical Field of the Invention

The invention belongs to the field of polymer chemistry, more precisely to the field of production of amino resins.

### Object of the Invention

An object of the present invention is a novel continuous process for the production of methylol melamines, especially of hexamethylol melamine and a mixture of higher methylol melamines, wherein a continuous synthesis from a formaldehyde solution and melamine is combined with a continuous drying of the resulting product mixture, whereby a product in the form of a powdery material with a low content of free formaldehyde and water is obtained.

Simultaneously, the invention also relates to a process for the production of highly etherified melamine resins based on hexamethylol melamine produced by the process of the present invention and to the use of hexamethylol melamine produced by the process of the present invention in the process for the production of highly etherified melamine resins. Particularly, it relates to a process for the production of hexamethoxymethylol melamine (HMMM), hexabutoxymethylol melamine (HBMM) and mixed ethers of C₁-C₅ alkohols on the basis of hexamethylol melamine produced by the process of the invention.

### Technical Problem and Prior Art

Among amino resins, especially among products on melamine basis, hexamethoxymethylol melamine (HMMM) has a special place. Due to its special structure it can be used in lacquer industry in water-soluble coatings and in coatings with solvents as well as in water-systems and in systems with little amounts of solvents (so-called "high solid" coating systems), and in rubber industry as an adhesion promoter.

From the literature (Fisch, Widmer and Gams, Helv. Chim. Acta 24, (1941), 303-319) it is known that the synthesis of hexamethoxymethylol melamine runs in such a manner that first melamine is reacted with an aqueous solution of excess formaldehyde, whereupon the obtained hexamethylol melamine is etherified with excess methanol.

The carrying out of the process on a laboratory scale using high excesses of reagents is not exceedingly demanding, yet at planning an economical industrial synthesis on an industrial scale we are faced with at least two great problems:
1. For binding six (6) molecules of formaldehyde on one (1) melamine molecule even in optimum synthesis conditions there is required an excess of an aqueous solution of formaldehyde at least in the range of 1-4 moles to 1 mole of melamine, which in practice means a ratio 7-10 moles of formaldehyde to 1 mole of melamine. Formaldehyde is commercially available in the form of aqueous solutions with concentrations of 30-55 wt.%. Hexamethylol melamine is practically insoluble in water. This means that as a reaction product hexamethylol melamine (HMM) is obtained in the form of a paste containing 40-70 wt. % of water and non-reacted formaldehyde. The disadvantages of such paste are its tendency toward hardening and its unfavourable rheological properties for mixing and further processing. Due to the high content of free formaldehyde it also has unfavourable ecological and safety characteristics.
2. Etherification of hexamethylol melamine with methanol is an equilibrium reaction. At the etherification of hexamethylol melamine with methanol an excess of methanol is necessary in any case in order to reach an equilibirum state near the etherification level 6, i.e. of all free -OH groups of hexamethylol melamine. In the etherification reaction water is formed. Water, which is brought into the system with hexamethylol melamine from an aqueous solution of formaldehyde, pushes the equilibrium of the chemical reaction to the left. This results in large excesses of methanol in the order of magnitude of 30-50 moles of methanol (instead of theoretical 6) to 1 mole of melamine being necessary in order to obtain hexamethoxymethylol melamine (HMMM). When other alcohols are used for etherification the situation is similar.

The production of hexamethylol melamine from paraformaldehyde and melamine does not give satisfactory results regarding the economy of the process and the quality of the product either. Formaldehyde in the form of paraformaldehyde is more expensive, the reaction nevertheless requires some addition of water and the resulting reaction mixture contains a large amount of free formaldehyde due to the necessary excesses and the incomplete depolymerization of paraformaldehyde.

A logical solution of this problem would be that after the first synthesis step hexamethylol melamine would be dried, whereupon a phase of etherification would follow. Yet it is known from industrial practice that the hexamethylol melamine paste formed in the reaction cannot be easily and economically dried on an industrial scale. Drying on open systems without stirring (e.g. on conveyor dryers etc.) is not practical because the velocity of diffusion of volatile components (water and formaldehyde) from interior to the surface is very low. Besides, the hexamethylol melamine paste sticks to most surfaces. Hexamethylol melamine is a thermally very sensitive material, its polymerization with degradation takes place already at 133°C. Such drying is also unfavourable from the ecological point of view since the products of drying, especially formaldehyde and methanol, may not be discharged into the environment.

Also drying with mixing such as in dryers with plough bars or spiral mixing means is not easily feasible. During the mixing the hexamethylol melamine paste becomes an increasingly dense and tough mass, which gradually requires increasingly bigger forces and an increasing input of mechanical energy for mixing. In dryers with stronger constructions, however, the input of mechanical energy for mixing results in local overheatings and thus in thermal polymerization and decomposition of hexamethylol melamine, which destroys the thermically sensitive product.

WO 99/08856 describes a continuous process for the production of amino- and/or phenoplasts as well as the use of the process for the production of melamine and phenol resins. In this document a process is described, wherein a pre-condensate solution produced in a first extruder and prepared according to the process known from EP 0 355 760 B1 is continuously, under the addition of additives, fed into a second extruder, wherein the product is dried and formed into the final form. At the end of the second extruder the product has a content of water of preferably 8-15 wt.%. The process deals with a partial drying and removal of gases or degassing since only a part of the pre-condensate solution produced in the first extruder is fed into the second extruder. From the specification of WO 99/08856 it is additionally evident that the invention mainly deals with the production of melamine resins with included fillers, especially as masses for pressing, whereat a polycondensation reaction and polymerization of melamine resins take place. The purpose of this invention was not to obtain as pure as possible monomeric methylol melamines with as low as possible content of the residual free formaldehyde. Preparation of methylol-functionalized melamine from formaldehyde and melamine is also described in WO 01/60882, whereas the invention of this application deals with crosslinker compositions consisting essentially of 50-95 wt.% monomeric C₁-C₈ alkoxymethyl melamine derivatives containing not more than about 0.2 wt.% imino groups.

The commonly known way of industrial production of hexamethoxymethylol melamine is as follows:

In a mixing reaction vessel melamine and an aqueous formaldehyde solution are reacted in a ratio melamine : formaldehyde from 1 : 6 to 1 : 10 at an alkaline pH. The resulting paste is mixed in the same or in another mixing vessel with a large excess of methanol (30-50 moles) and etherified at an acidic pH. The excessive methanol, water and formaldehyde are usually removed by distillation. There remains hexamethoxymethylol melamine, which still has a certain content of residual formaldehyde and water and therefore usually has to be further purified.

As a rule, batch yields do not reach one fourth of the reaction mixture at etherification, thus the productivity is relatively low and per one unit of the product at least three units of side products are formed, which have to be recycled or ecologically destroyed, which is connected with much expense.

### Solution to the Technical Problem

The task of the present invention was to develop an economical continuous process for the production of methylol melamines, especially hexamethylol melamine and a mixture of higher methylol melamines, wherein a continuous synthesis of hexamethylol melamine or of a mixture of higher methylol melamines from a formaldehyde solution and melamine at an alkaline pH in a continuous mixing reactor or extruder would be combined with a continuous drying of the resulting product mixture in continuous mixing vacuum dryer. Particularly, the task of the present invention was to develop a continuous process, whereby the problem of drying the product of the reaction between melamine and formaldehyde in a continuous manner would be solved and at the same time a high quality product with a small content of residual free formaldehyde and water would be obtained. The task of the invention was also to develop a process, whereat the volatile products of the drying would represent low ecological damage and be recycable.

At the same time the task of the present invention was to develop a process enabling a highly productive and environment-friendly synthesis of highly etherified melamine resins, especially hexamethoxymethylol melamine (HMMM), hexabutoxymethylol melamine (HBMM) or mixed ethers of C₁-C₅ alcohols based on hexamethylol melamine, whereat final articles of high quality and with a low content of free monomers, especially formaldehyde would be obtained.

The novel process for production of methylol melamines according to the invention is carried out in such a manner that:
a) a solution of formaldehyde, a solution of an alkalizing agent and water in a proper ratio ranging from 100 : 0.01 : 0 to 100 : 5 : 100 in order to obtain a solution with an alkaline pH and an appropriate concentration are fed into an extruder or a continuous mixing reactor, into which melamine in a ratio melamine : formaldehyde from 1 : 4 to 1 : 40 is continuously added, whereupon under mixing a reaction takes place at controlled conditions of temperature and pH so that a product mixture in the form of a paste is obtained at the end of the reactor, and
b) the resulting product mixture in the form of a paste is continuously fed into a continuous mixing vacuum dryer shaped in such a manner that the paste turns into the tough phase only in a narrow area and that back-mixing with the already dried final product in powder form takes place so that a granulation of the tough material and a further drying of the product mixture in the form of a granulate are achieved and the final product of the drying is a dry powdery material hexamethylol melamine or a mixture of higher methylol melamines, whereat the drying is carried out at a temperature in a range from 50 to 120°C and at a pressure from 30 to 200 mbar, and
c) whereat the volatile products of the drying formed in the step b) are condensed on a condenser so that they are obtained in the form of an aqueous solution predominantly containing formaldehyde.

Into the reaction mixture of step a) of the above process, melamine can be fed in a ratio melamine : formaldehyde from 1 : 4 to 1 : 40, preferably in a ratio melamine : formaldehyde from 1 : 4 to 1 : 10.

The alkalizing agent used in step a) of the above process can be an alkali hydroxide, alkali hydrogen carbonate or alkali carbonate, e.g. sodium hydroxide, potassium hydroxide, potassium carbonate or potassium hydrogen carbonate, preferably sodium hydroxide.

The reaction in step a) of the above process may take place at a pH from 8 to 11, preferably at a pH from 9.5 to 10 and at a temperature from 20 to 100°C, preferably from 40 to 95°C, most preferably from 55 to 85°C.

An average holding time in step a) of the process is from 15 to 35 minutes.

The product mixture of step a) of the process according to the invention, which is in the form of a paste, contains a mixture of precipitated higher methylol melamines or precipitated hexamethylol melamine, water and excess formaldehyde and smaller amounts of other reaction components.

The drying of the product mixture in step b) of the process according to the invention is carried out at a temperature from 50 to 120°C, preferably at a temperature from 80 to 120°C and at a pressure from 30 to 200 mbar, preferably at about 100 mbar.

Depending on the ratio melamine : formaldehyde and on the reaction conditions in step a) of the process according to the invention, either hexamethylol melamine or a mixture of higher methylol melamines can be produced by the process for production of methylol melamines according to the invention.

As a suitable continuous mixing reactor in step a) of the process according to the invention there can be used e.g. a reactor of the manufacturer LIST AG, Switzerland, e.g. LIST ORP or LIST CRP, or an extruder e.g. a twin-screw extruder of the Theyson ZSK type.

As a suitable continuous mixing vacuum dryer in step b) of the process according to the invention there can be used e.g. a reactor of the manufacturer LIST AG, Switzerland, e.g. LIST DTB.

According to one embodiment of the invention hexamethylol melamine is produced according to the above novel process. In more detail, the process for the production of hexamethylol melamine is carried out in such manner that a solution of formaldehyde, a sodium hydroxide solution and water in a proper ratio ranging from 100 : 1 : 1 to 100 : 5 : 100 so that a solution with an alkaline pH (pH = 8-11, preferably 9.5-10) and appropriate concentration is obtained, are fed with dosing pumps (e.g. of the manufacturer Brand & Luebe) through static mixers into an extruder or a continuous mixing reactor (such as e.g. LIST ORP or LIST CRP), whereto melamine in a ratio melamine : formaldehyde from 1 : 6 to 1 : 10 is continuously fed. In the reactor a reaction between melamine and formaldehyde takes place under controlled conditions of temperature in a range from 20-100°C, preferably 40-95°C, most preferably 55-85°C, and at pH from 8 to 11, preferably at a pH from 9.5 to 10, first as a dissolving of melamine in the aqueous solution of formaldehyde, whereby predominantly mono-, di- and trimethylol melamines are formed, and subsequently as a precipitation reaction, whereby higher methylol melamines and finally hexamethylol melamine are formed. The average holding time is from 15 to 35 minutes. At the end of the reactor a product mixture containing precipitated hexamethylol melamine, water and excessive formaldehyde and smaller amounts of other reaction components is obtained in the form of a more or less thick paste depending on, in particular, the contents of water and non-reacted formaldehyde.

The resulting paste is continuously fed into a continuous mixing vacuum dryer (such as e.g. LIST DTB), wherein it is continuously dried. The paste first thickens and then turns into a tough form, which at batch drying would require very high mechanical forces for mixing and kneading, which would consequently lead to local overheatings and thereby to a decomposition of the hexamethylol melamine paste. In the novel process according to the present invention it is achieved by the use of a specially designed continuous mixing vacuum dryer so that the tough phase is only formed in a narrow area and that back-mixing with the already dried final product in powder form takes place so that a granulation of the tough material and a further drying of hexamethylol melamine in the form of a granulate are achieved, which prevents extreme mechanical stresses of the dryer and an over-input of mechanical energy and thus a local overheating and decomposition of hexamethylol melamine. The drying is carried out at temperatures from 50 to 120°C, preferably from 80 to 120°C and at a pressure from 30 to 200 mbar, preferably at about 100 mbar. By means of special geometry of the mixing dryer and the back-mixing of the already dried product hexamethylol melamine in powder form, a better drying and a better evaporation of the volatile products of drying, especially of formaldehyde and water are achieved.

The volatile products of drying are condensed on a condenser in such a manner that they are obtained in the form of an aqueous solution that predominantly contains formaldehyde and is suitable for recycling.

Hexamethylol melamine produced according to one preferred embodiment of the process according to the invention is a powdery material, which distinguishes itself in a low content of free formaldehyde, which is under 3 wt.%, and a low content of water which is under 3 wt. %.

In an analogous manner as described above for the production of hexamethylol melamine, by the use of an appropriate ratio of melamine : formaldehyde and of appropriate reaction conditions also a mixture of higher methylol melamines can be produced.

The mixture of higher methylol melamines produced according to a further preferred embodiment of the process according to the invention is a powdery material, which distinguishes itself in a low content of free formaldehyde, which is under 0.8 wt. %, and in the content of water being under 5 wt.%.

Higher methylol melamines are suitable for the preparation of acidic colloids serving as wet-strength agents in paper manufacture. They are also useful as an additive to pressing masses on the basis of other aminoplasts and phenoplasts or as a raw material for the production of other etherified melamine resins.

By the novel process for the production of methylol melamines according to the invention combining a continuous synthesis from a formaldehyde solution and melamine at an alkaline pH and a continuous drying of the formed product mixture a good productivity is achieved. Simultaneously, by the process according to the invention also problems connected with ecologically problematical waste products of the reaction are successfully solved and costs connected with their ecological destruction are avoided. The waste products obtained in the process according to the invention are suitable for recycling.

Hexamethylol melamine obtained by the process of the invention is particularly suitable for use in a process of production of highly etherified melamine resins. Due to the low content of water, hexamethylol melamine obtained by the process of the invention enables a more efficient etherification with alcohol and thus a high productivity of the process of the production of highly etherified melamine resins. As it has a low content of free formaldehyde, also the final products of the etherification process have a very low content of free formaldehyde.

A process of production of highly etherified melamine resins, which is a further object of the present invention, is carried out in such a manner that hexamethylol melamine produced by the process of the invention is continuously or batchwise etherified with C₁-C₅ alcohols in a ratio hexamethylol melamine : C₁-C₅ alcohol from 1 : 8 to 1 : 30 at controlled temperature and pH conditions, which is followed by neutralization and removal of volatile components with vacuum distillation.

The preferred C₁-C₅ alcohol for etherification is methanol.

The above process is conducted at a temperature in a range from 20 to 100°C, preferably at a temperature from 45-65°C and in a pH range from 1.5 to 4.0.

As an agent for adjusting the acidic pH there can be used nitric, formic, phosphoric, sulfuric or hydrochloric acid and preferably nitric acid is used.

The products of the etherification are highly etherified melamine resins, especially hexamethoxymethylol melamine (HMMM), hexabutoxymethylol melamine (HBMM) and mixed ethers of C₁-C₅ alcohols based on HMM, preferably hexamethoxymethylol melamine. The products of the process of production of highly etherified melamine resins according to the invention have a low content of free formaldehyde. Thus, hexamethoxymethylol melamine obtained by the process of the invention has a free formaldehyde content under 0.08 wt. %.

The productivity of this process is three times higher than the productivity of the process of etherification of wet hexamethylol melamine (with a water content of about 50 wt.%). The process not only excells in high productivity per one unit of reactor volume, but also makes possible the production of quality highly etherified melamine resins, especially hexamethoxymethylol melamine, wherein in order to achieve a high quality of the products, particularly with regard to a low content of free monomers especially formaldehyde, no additional processes for removal of free formaldehyde (e.g. washing or additional etherification) are necessary.

As the process for the production of hexamethylol melamine according to the invention is continuous, it makes possible a continuous production of highly etherified melamine resins, which is a further object of the present invention.

The continuous process for the production of highly etherified melamine resins, preferably hexamethoxymethylol melamine, takes place in such a manner that the continuous production of hexamethylol melamine according to the invention is combined with the continuous etherification of the obtained hexamethylol melamine with C₁-C₅ alcohols, preferably methanol in a ratio hexamethylol melamine : C₁-C₅ alcohol from 1 : 8 to 1 : 30, at controlled temperature and pH conditions, which is followed by neutralization and removal of volatile components represented by excessive alcohol, water and formaldehyde by vacuum distillation.

### Examples

### Production of hexamethylol melamine

### Example 1

By a multihead dosing pump (e.g. by the manufacturer Brand & Luebe), a solution of formaldehyde (45 %), a solution of sodium hydroxide (25 %), and water in a ratio CH₂O : NaOH : H₂O = 100 : 1 : 10, so that the solution had a pH in the range 9.5-10, were fed through static mixers into a continuous mixing reactor LIST ORP, whereto melamine was continuously added so that the ratio melamine to formaldehyde M/F = 1 : 7 was obtained. In the reactor a reaction between melamine and formaldehyde took place under controlled conditions at the temperature of 70°C and under mixing with an average holding time of 15-30 minutes, at first as the dissolving of melamine in the aqueous solution of formaldehyde, wherein predominantly mono-, di- and trimethylol melamines were formed, and then as precipitation reaction, wherein higher methylol melamines and finally hexamethylol melamine (HMM) were formed.

At the end of the reactor a mixture of the precipitated hexamethylol melamine, water and excessive formaldehyde and of smaller amounts of other reaction components in the form of a paste was continuously led away into a continuous mixing vacuum dryer LIST DTB, wherein it was continuously dried at 100°C and 100 mbar. The volatile drying products were condensed on a condenser and obtained in the form of an aqueous solution containing predominantly formaldehyde, which was suitable for recycling.

The product of drying was a dry powder hexamethylol melamine with a content of bound formaldehyde of 6.0 moles to 1 mole of melamine, with a content of free formaldehyde under 2 wt. % and a water content of 1-3 wt. %.

### Example 2

The same steps as in the Example 1 were performed with the exception that all components were not fed into LIST ORP but into an extruder, most appropriately into a twin screw extruder (e.g. Theyson ZSK), the holding time had to be 15-30 minutes as well and the temperature was controlled at 70°C. The process of drying was the same as in the Example 1.

The product of drying was the same as in the Example 1.

### Example 3

By a multihead dosing pump (e.g. by the manufacturer Brand & Luebe) a solution of formaldehyde (45 %), a solution of sodium hydroxide (25 %), and water in a ratio CH₂O : NaOH : H₂O = 100 : 1 : 1 so that the solution had a pH in the range from 9.5-10, were fed through static mixers into a continuous mixing reactor LIST ORP, whereto melamine was continuously added so that the ratio of melamine to formaldehyde M/F = 1 : 9 was obtained. In the reactor a reaction between melamine and formaldehyde took place under controlled conditions at the temperature of 70°C and under mixing with an average holding time of 15-30 minutes, at first as the dissolving of melamine in the aqueous solution of formaldehyde, wherein predominantly mono-, di- and trimethylol melamines were formed, and then as a precipitation reaction, wherein higher methylol melamines and finally hexamethylol melamine were formed.

At the end of the reactor a mixture of the precipitated hexamethylol melamine, water and excessive formaldehyde and of smaller amounts of other reaction components in the form of a paste was continuously led away into a continuous mixing vacuum dryer LIST DTB, where it was continuously dried at 120°C and 100 mbar. The volatile products of drying were condensed on a condenser and obtained in the form of an aqueous solution containing predominantly formaldehyde, which was suitable for recycling.

The product of drying was a dry powder hexamethylol melamine with a content of bound formaldehyde 6.0 moles to 1 mole of melamine, with a content of free formaldehyde under 3 wt. % and a water content of 1-3 wt. %.

### Example 4

By a multihead dosing pump (e.g. by the manufacturer Brand & Luebe), a solution of formaldehyde (45 %), a solution of sodium hydroxide (25 %), and water in a ratio CH₂O : NaOH : H₂O = 100 : 1 : 1, so that the solution had a pH in the range 9.5-10, were fed through static mixers into a continuous mixing reactor LIST ORP, whereto melamine was continuously added so that the ratio melamine to formaldehyde M/F = 1 : 7 was obtained. In the reactor a reaction between melamine and formaldehyde took place under controlled conditions at the temperature of 85°C and under mixing with an average holding time of 10-20 minutes, at first as the dissolving of melamine in the aqueous solution of formaldehyde, wherein predominantly mono-, di- and trimethylol melamines were formed, and then as precipitation reaction, wherein higher methylol melamines and finally hexamethylol melamine were formed.

At the end of the reactor a mixture of the precipitated hexamethylol melamine, water and excessive formaldehyde and of smaller amounts of other reaction components in the form of a thick paste was continuously led away into a continuous mixing vacuum dryer LIST DTB, wherein it was continuously dried at 100°C and 100 mbar. The volatile products of drying were condensed on a condenser and obtained in the form of an aqueous solution containing predominantly formaldehyde, which was suitable for recycling.

The product of drying was a dry powder hexamethylol melamine with a content of bound formaldehyde of 6.0 moles to 1 mole of melamine, with a content of free formaldehyde under 3 wt. % and a water content of 1-3 wt. %.

### Comparative Example 5

By a multihead dosing pump (e.g. by the manufacturer Brande & Luebe), a solution of formaldehyde (45 %), a solution of sodium hydroxide (25 %), and water in a ratio CH₂O : NaOH : H₂O = 100 : 1 : 10, so that the solution had a pH in the range 9.5-10, were fed through static mixers into a continuous mixing reactor LIST ORP, whereto melamine was continuously added so that the ratio melamine to formaldehyde M/F = 1 : 7 was obtained. In the reactor a reaction between melamine and formaldehyde took place under controlled conditions at the temperature of 70°C and under mixing with an average holding time 15-30 minutes, at first as the dissolving of melamine in the aqueous solution of formaldehyde, wherein predominantly mono-, di- and trimethylol melamines were formed, and then as precipitation reaction, wherein higher methylol melamines (MM) and finally hexamethylol melamine (HMM) were formed. The reaction resulted in a mixture of precipitated hexamethylol melamine, water, excessive formaldehyde and of smaller amouts of other reaction compontents in the form of a paste. The product of a reaction, the formed HMM paste, had a content of bound formaldehyde of 6.0 moles to 1 mole of melamine, a content of free formaldehyde under 8 % and a water content of 40 -50 %. When left standing the paste hardened into a white mass.

### Production of a mixture of higher methylol melamines

### Example 6

By a multihead dosing pump (e.g. by the manufacturer Brand & Luebe), a solution of formaldehyde (45 %), a solution of sodium hydroxide (25 %), and water in a ratio CH₂O : NaOH : H₂O = 100 : 1 : 1, so that the solution had a pH in the range 9.5-10, were fed through static mixers into a continuous mixing reactor LIST ORP, whereto melamine was continuously added so that the ratio melamine to formaldehyde M/F = 1 : 4.4 was obtained. In the reactor a reaction between melamine and formaldehyde took place under controlled conditions at the temperature of 55°C and under mixing with an average holding time of 20-30 minutes, at first as the dissolving of melamine in the aqueous solution of formaldehyde, wherein predominantly mono-, di- and trimethylol melamines were formed, and then as precipitation reaction, wherein higher methylol melamines were formed.

At the end of the reactor a mixture of the precipitated hexamethylol melamine, water and excessive formaldehyde and of smaller amounts of other reaction components in the form of a thin paste was continuously led away into a continuous mixing vacuum dryer LIST DTB, wherein it was continuously dried at 80°C and 100 mbar. The volatile drying products were condensed on a condenser and obtained in the form of an aqueous solution containing predominantly formaldehyde, which was suitable for recycling.

The product of drying was a dry powder MM with a content of bound formaldehyde of 4.0-4.2 moles to 1 mole of melamine, with a content of free formaldehyde under 0.8 % and a water content of 1-5 %.

The product is useful e.g. for the manufacture of acidic colloids serving as wet-strength agents in paper manufacturing or as a raw material for manufacturing other etherified melamine resins.

### Example 7

By a multihead dosing pump (e.g. by the manufacturer Brand & Luebe), a solution of formaldehyde (45 %), a solution of sodium hydroxide (25 %), and water in a ratio CH₂O : NaOH : H₂O = 100:1:1, so that the solution had a pH in the range 9.5-10, were fed through static mixers into a continuous mixing reactor LIST ORP, whereto melamine was continuously added so that a ratio melamine to formaldehyde M/F = 1 : 5.5 was obtained. In the reactor a reaction between melamine and formaldehyde took place under controlled conditions at the temperature of 65°C and under mixing with an average holding time of 20-35 minutes, at first as the dissolving of melamine in the aqueous solution of formaldehyde, wherein predominantly mono-, di- and trimethylol melamines were formed, and then as precipitation reaction, wherein higher methylol melamines were formed.

At the end of the reactor a mixture of precipitated higher methylol melamines, water and excessive formaldehyde and of smaller amounts of other reaction components in the form of a thin paste was continuously led away into a continuous mixing vacuum dryer LIST DTB, wherein it was continuously dried at 80°C and 100 mbar abs. The volatile products of drying were condensed on a condenser and obtained in the form of an aqueous formaldehyde solution, which was suitable for recycling.

The product of drying was dry powder MM with a content of bound formaldehyde of 5.0-5.2 moles to 1 mole of melamine, with a content of free formaldehyde under 0.8 % and a water content of 1-5 %.

The product is useful e.g. as an additive to pressing masses on the basis of other aminoplasts or phenoplasts or as a raw material for manufacturing other etherified melamine resins.

### Production of hexamethoxymethylol melamine

### Example 8

Dry hexamethylol melamine from Example 1 was dosed into a suitable mixing vessel equipped with heating, cooling and mixing equipment. To 1 mole of hexamethylol melamine 12 moles of methanol were added, the pH was set to 1.5-1.9 with nitric acid and it was etherified at 45°C for 1.5 hours. Then the reaction mixture was neutralized with a NaOH solution and the volatile components were vacuum-distilled off. The properties of the product hexamethoxymethylol melamine are given in Table 1.

### Example 9

Dry hexamethylol melamine from Example 1 was dosed into a suitable mixing vessel equipped with heating, cooling and mixing equipment. To 1 mole of hexamethylol melamine 9 moles of methanol were added, the pH was set to 3.6-3.8 with nitric acid and it was etherified at 55°C for 1.5 hours. Then the reaction mixture was neutralized with a NaOH solution and the volatile components were vacuum-distilled off. The properties of the product hexamethoxymethylol melamine are given in Table 1.

### Comparative Example 10

Hexamethylol melamine from the Comparative Example 5 was dosed into a suitable mixing vessel equipped with heating, cooling and mixing equipment. To 1 mole of hexamethylol melamine 12 moles of methanol were added, the pH was set to 3.6-3.8 with nitric acid and it was etherified at 55°C for 1.5 hours. Then the reaction mixture was neutralized with a NaOH solution and the volatile components were vacuum-distilled off. The properties of the product hexamethoxymethylol melamine (HMMM) are given in Table 1.

### Comparative Example 11

Hexamethylol melamine from the Comparative Example 5 was dosed into a suitable mixing vessel equipped with heating, cooling and mixing equipment. To 1 mole of hexamethylol melamine 30 moles of methanol were added, the pH was set to 1.5-1.9 with nitric acid and it was etherified at 45°C for 1.5 hours. Then the reaction mixture was neutralized with a NaOH solution and the volatile components were vacuum-distilled off. The properties of the product hexamethoxymethylol melamine (HMMM) are given in Table 1.

### Example 12

Dry hexamethylol melamine from Example 1 was dosed into a suitable mixing vessel equipped with heating, cooling and mixing equipment. To 1 mole of hexamethylol melamine 30 moles of methanol were added, the pH was set to 1.5-1.9 with nitric acid and it was etherified at 45°C for 1.5 hours. Then the reaction mixture was neutralized with a NaOH solution and the volatile components were vacuum-distilled off. The properties of the product hexamethoxymethylol melamine are given in Table 1.

**Table 1: Properties of HMMM products**

| Example No. | Portion of methanol (moles of methanol/ 1 mol of melamine) | Viscosity (mPa.s) | Dry substance (wt.%) | Free formaldehyde (wt.%) | B - time (gelling time) (s) |
|---|---|---|---|---|---|
| 8 | 12 | 2740 | 96 | 0.04 | 355 |
| 9 | 9 | 5600 | 95.6 | 0.07 | 150 |
| 10 | 12 | 58000 | 92 | 2.1 | 80 |
| 11 | 30 | 11000 | 93 | 1.1 | 130 |
| 12 | 30 | 1560* | 96.5 | 0.03 | 412 |

| | | | | | |
|---|---|---|---|---|---|
| * the product crystalizes (it contains too much monomeric HMM) | | | | | |

## Claims

1. A process for production of methylol melamines, **characterized in that**
a) a solution of formaldehyde, a solution of an alkalizing agent and water in a proper ratio ranging from 100 : 0.01 : 0 to 100 : 5 : 100 in order to obtain a solution with alkaline pH and appropriate concentration are fed into an extruder or a continuous mixing reactor, into which melamine in a ratio melamine: formaldehyde from 1 : 4 to 1 : 40 is continuously added, whereupon under mixing a reaction takes place at controlled conditions of temperature and pH so that, a product mixture in the form of a paste is obtained at the end of the reactor, and
b) the resulting product mixture in the form of a paste is continuously fed into a continuous mixing vacuum dryer shaped in such a manner that the paste turns into the tough phase only in a narrow area and that back-mixing with the already dried final product in powder form takes place so that a granulation of the tough material and a further drying of the product mixture in the form of a granulate are achieved and the final product of the drying is a dry powdery material hexamethylol melamine or a mixture of higher methylol melamines, whereat the drying is carried out at a temperature in a range from 50 to 120°C and at a pressure from 30 to 200 mbar, and
c) whereat the formed volatile products of the drying formed in the step b) are condensed on a condenser so that they are obtained in the form of an aqueous solution predominantly containing formaldehyde.

2. A process according to claim 1, **characterized in that** into the reaction mixture in the step a) of the process according to claim 1 melamine is fed in a ratio melamine : formaldehyde from 1 : 4 to 1 : 10.

3. A process according to claim 1 or 2, **characterized in that** as the alkalizing agent in the step a) of the process according to claim 1 an alkali hydroxide, alkali hydrogen carbonate or alkali carbonate, preferably sodium hydroxide is used.

4. A process according to any of the claims 1 to 3, **characterized in that** the reaction in the step a) of the process according to claim 1 takes place at a pH from 8 to 11, preferably at a pH from 9.5 to 10.

5. A process according to any of the claims 1 to 4, **characterized in that** the reaction in the step a) of the process according to claim 1 takes place at a temperature from 20 to 100 °C, preferably from 40 to 95 °C, most preferably from 55 to 85°C.

6. At process according to any of the claims 1 to 5, **characterized in that** the drying in the step b) of the process according to claim 1 takes place at a temperature from 50 to 120°C, preferably at a temperature from 80 to 120°C, and at a pressure from 30 to 200 mbar, preferably at about 100 mbar.

7. A process according to any of the claims 1 to 6, **characterized in that** the product of the process is hexamethylol melamine in powder form.

8. A process according to claim 7, **characterized in that** the product of the process is hexamethylol melamine having a free formaldehyde content under 3 wt.% and a water content under 3 wt.%.

9. A process according to any of the claims 1 to 6, **characterized in that** the product , of the process is a mixture of higher methylol melamines in powder form.

10. A process according to claim 9, **characterized in that** the product is a mixture of higher methylol melamines having a free formaldehyde content under 0.8 wt.% and a water content under 5 wt.%.

11. A process of production of highly etherified melamine resins, **characterized in that** hexamethylol melamine produced by a process according to any of the claims 1 to 8 is continuously or batchwise etherified with C₁-C₅ alcohols in a ratio hexamethylol melamine : C₁-C₅ alcohol from 1 : 8 to 1 : 30 under controlled conditions of temperature and pH, followed by neutralization and removal of volatile components by vacuum distillation.

12. A process of production of highly etherified melamine resins, **characterized in that** it is carried out continuously in such a manner that a continuous process according to any of the claims 1 to 8 is combined with a continuous etherification of hexamethylol melamine obtained by a process according to any of the claims 1 to 8, with C₁-C₅ alcohols in a ratio hexamethylol melamine : C₁-C₅ alcohol from 1 : 8 to 1 : 30 under controlled conditions of temperature and pH, followed by neutralization and removal of volatile components by vacuum distillation.

13. A process according to claim 11 or 12, **characterized in that** the C₁-C₅ alcohol is methanol.

14. A process according to any of the claims 11 to 13, **characterized in that** the product of the process is hexamethoxymethylol melamine, hexabutoxymethylol melamine or mixed ethers of C₁-C₅ alcohols on the basis of hexamethylol melamine, preferably hexamethoxymethylol melamine.

15. A process according to any of the claims 11 to 14, **characterized in that** the product is hexamethoxymethylol melamine having a free formaldehyde content under 0.08 wt.%.

## Patentansprüche

1. Verfahren zur Erzeugung von Methylolmelaminen, **dadurch gekennzeichnet, dass**
a) eine Lösung Formaldehyd, eine Lösung eines Alkalisierungsmittels und in einem zum Erhalten einer Lösung mit alkalischem pH und einer geeigneten Konzentration geeigneten Verhältnis im Bereich von 100 : 0,01 : 0 zu 100 : 5 : 100 einem Extruder oder einem kontinuierlichen Mischreaktor zugeführt werden, zu welchem Melamin in einem Verhältnis Melamin : Formaldehyd von 1 : 4 bis 1 : 40 kontinuierlich hinzugegeben wird, wobei unter Mischen eine Reaktion unter kontrollierten Temperatur- und pH-Bedingungen derart stattfindet, dass eine Produktmischung in der Form einer Paste am Ende des Reaktors erhalten wird, und
b) die resultierende Produktmischung in der Form einer Paste kontinuierlich in einen Vakuumtrockner mit kontinuierlichem Mischer zugeführt wird, welcher in einer solchen Art und Weise ausgestaltet ist, dass sich die Paste nur in einem schmalen Bereich in eine zähe Phase umwandelt und dass die Rückmischung mit dem schon getrockneten Endprodukt in Pulverform derart stattfindet, dass eine Granulierung des zähen Materials und eine weitere Trocknung der Produktmischung in der Form eines Granulats erzielt wird und das Endprodukt des Trocknens ein trockenes pulvriges Material aus Hexamethylolmelamin oder einer Mischung aus höheren Methylolmelaminen ist, wobei die Trocknung bei einer Temperatur in einem Bereich von 50 bis 120°C und einem Druck von 30 bis 200 mbar durchgeführt wird, und
c) wobei die geformten flüchtigen Produkte des im Schritt b) erzeugten Trocknens auf einem Kühler derart kondensiert werden, dass sie in der Form einer überwiegend Formaldehyd enthaltenden wässrigen Lösung erhalten werden.

2. Verfahren gemäß Anspruch 1, **dadurch gekennzeichnet, dass** in die Reaktionsmischung in dem Schritt a) des Verfahrens gemäß Anspruch 1 Melamin in einem Verhältnis Melamin : Formaldehyd von 1 : 4 bis 1 : 10 zugeführt wird.

3. Verfahren gemäß Anspruch 1 ober 2, **dadurch gekennzeichnet, dass** als das Alkalisierungsmittel in dem Schritt a) des Verfahrens gemäß Anspruch 1 ein Alkalihydroxid, Alkalyhydrogenkarbonat oder Alkalicarbonat, bevorzugt Natriumhydroxid, eingesetzt wird.

4. Verfahren gemäß irgendeinem der Ansprüche 1 bis 3, **dadurch gekennzeichnet, dass** die Realtion in dem Schritt a) des Verfahrens gemäß Anspruch 1 bei einem pH von 8 bis 11, bevorzugt bei einem von pH 9,5 bis 10 stattfindet.

5. Verfahren gemäß irgendeinem der Ansprüche 1 bis 4, **dadurch gekennzeichnet, dass** die Reaktion in dem Schritt a) des Verfahrens gemäß Anspruch 1 bei einer Temperatur von 20 bis 100°C, bevorzugt von 40 bis 95°C und am meisten bevorzugt von 55 bis 85°C stattfindet.

6. Verfahren gemäß irgendeinem der Ansprüche 1 bis 5, **dadurch gekennzeichnet, dass** die Trocknung in dem Schritt b) es Verfahrens gemäß Anspruch 1 bei einer Temperatur von 50 bis 120°C, bevorzugt bei einer Temperatur von 80 bis 120°C, und bei einem Druck von 30 bis 200 mbar, bevorzugt bei ca. 100 mbar, stattfindet.

7. Verfahren gemäß irgendeinem der Ansprüche 1 bis 6, **dadurch gekennzeichnet, dass** das Produkt des Verfahrens Hexamethylolmelamin in Pulverform ist.

8. Verfahren gemäß Anspruch 7, **dadurch gekennzeichnet, dass** das Produkt des Verfahrens Hexamethylolmelamin mit einem Formaldehydgehalt unter 3 Gew.% und einem Wassergehalt unter 3 Gew.% ist.

9. Verfahren gemäß irgendeinem der Ansprüche 1 bis 6, **dadurch gekennzeichnet, dass** das Produkt des Verfahrens eine Mischung aus höheren Methylolmelaminen in Pulverform ist.

10. Verfahren gemäß Anspruch 9, **dadurch gekennzeichnet, dass** das Produkt eine Mischung aus höheren Methylolmelaminen mit einem freien Formaldehydgehalt unter 0,8 Gew.% und einem Wassergehalt unter 5 Gew.% ist.

11. Verfahren zur Erzeugung eines hochveretherten Melaminharzes, **dadurch gekennzeichnet, dass** das durch ein Verfahren gemäß irgendeinem der Ansprüche 1 bis 8 erzeugte Hexamethylolmelamin kontinuierlich oder absatzweise mit C₁-C₅-Alkoholen in einem Verhältnis Hexamethylolmelamin : C₁-C₅-Alkohol von 1 : 8 bis 1 : 30 unter kontrollierten Temperatur- und pH-Bedingungen verethert wird, gefolgt von einer Neutralisation und einer Entfernung der flüchtigen Komponenten mittels Vakuumdestillation.

12. Verfahre zur Erzeugung von hochveretherten Melaminharzen, **dadurch gekennzeichnet, dass** es kontinuierlich in einer solchen Art und Weise durchgeführt wird, dass ein kontinuierliches Verfahren gemäß irgendeinem der Ansprüche 1 bis 8 mit einer kontinuierlichen Veretherung von durch ein Vorfahren gemäß irgendeinem der Ansprüche 1 bis 8 erhältlichem Hexamethylolmelamin mit C₁-C₅-Alkoholen in einem Verhältnis Hexamethylolmelamin : C₁-C₅-Alkohol von 1 : 8 bis 1 : 30 unter kontrollierten Temperatur- und pH-Bedingungen kombiniert wird, gefolgt von einer Neutralisation und einer Entfernung von flüchtigen Komponenten mittels Vakuumdestillation.

13. Verfahren gemäß Anspruch 11 oder 12, **dadurch gekennzeichnet, dass** der C₁-C₅-Alkohol Methanol ist.

14. Verfahren gemäß irgendeinem der Ansprüche 11 bis 13, **dadurch gekennzeichnet, dass** das Produkt des Verfahrens Hexamethoxymethylolmelamin, Hexabutoxymethylolmelamin oder gemischte Ether aus C₁-C₅-Alkoholen auf der Basis von Hexamethylolmelamin, bevorzugt Hexamethoxymethylolmelamin, ist.

15. Verfahren gemäß irgendeinem der Ansprüche 11 bis 14, **dadurch gekennzeichnet, dass** das Produkt Hexamethoxymethylolmelamin mit einem freien Formaldehydgehalt unter 0,08 Gew.% ist.

## Revendications

1. Procédé de production de méthylolmélamines, **caractérisé en ce que**
a) une solution de formaldéhyde, une solution d'un agent alcalisant et de l'eau, selon un rapport approprié compris entre 100:0,01:0 et 100:5:100, de façon à obtenir une solution avec un pH alcalin et une concentration appropriée, sont amenées dans une extrudeuse ou dans un réacteur de mélange en continu dans lequel on ajoute en continu de la mélamine selon un rapport de mélamine:formaldéhyde de 1:4 à 1:40, après quoi, par mélange, une réaction apparaît dans des conditions maîtrisées de température et de pH, de sorte qu'on obtient un mélange de produits sous la forme d'une pâte à l'extrémité du réacteur, et
b) le mélange de produits résultant sous la forme d'une pâte est amené en continu dans un séchoir sous vide de mélange en continu, façonné d'une manière telle que la pâte passe en phase dure seulement dans une zone étroite et qu'un mélange en retour avec le produit final déjà séché à l'état pulvérulent apparaît, de sorte qu'on obtient une granulation de la matière dure et un séchage supplémentaire du mélange de produits sous la forme d'un granulat, et le produit final du séchage est une matière pulvérulente sèche d'hexaméthylolmélamine ou un mélange de méthylolmélamines supérieures, dans lequel le séchage est effectué à une température dans une plage de 50 à 120 °C et à une pression de 30 à 200 mbar, et
c) dans lequel les produits volatils formés du séchage, formés dans l'étape b), sont condensés dans un condenseur, de sorte qu'on les obtient sous la forme d'une solution aqueuse contenant de façon prédominante du formaldéhyde.

2. Procédé selon la revendication 1, **caractérisé en ce que**, dans le mélange réactionnel dans l'étape a) du procédé selon la revendication 1, de la mélanine est amenée selon un rapport de mélamine:formaldéhyde de 1:4 à 1:10.

3. Procédé selon la revendication 1 ou 2, **caractérisé en ce qu'**on utilise comme agent alcalisant, dans l'étape a) du procédé selon la revendication 1, un hydroxyde alcalin, un hydrogénocarbonate alcalin ou un carbonate alcalin, de préférence de l'hydroxyde de podium.

4. Procédé selon l'une quelconque revendication 1 à 3, **caractérisé en ce que** la réaction dans l'étape a) du procédé selon la revendication 1 se déroule à un pH de 8 à 11, de préférence à un pH de 9, 5 à 10.

5. Procédé selon l'une quelconque des revendications 1 à 4, **caractérisé en ce que** la réaction dans l'étape a) du procédé selon la revendication 1 se déroule à une température de 20 à 100 °C, avantageusement de 40 à 95 °C, de préférence de 55 à 85 °C.

6. Procédé selon l'une quelconque des revendications 1 à 5, **caractérisé en ce que** le séchage dans l'étape b) du procédé selon la revendication 1 se déroule à une température de 50 à 120 °C, de préférence à une température de 80 à 120 °C, et à une pression de 30 à 200 mbar, de préférence à environ 100 mbar.

7. Procédé selon l'une quelconque des revendications 1 à 6, **caractérisé en ce que** le produit du procédé est de l'hexaméthylolmélamine à l'état pulvérulent.

8. Procédé selon la revendication 7, **caractérisé en ce que** le produit du procédé est de l'hexaméthylolmélamine ayant une teneur en formaldéhyde libre inférieure à 3 % en poids et une teneur en eau inférieure à 3 % en poids.

9. Procédé selon l'une quelconque des revendications 1 à 6, **caractérisé en ce que** le produit du procédé est un mélange de méthylolmélamines supérieures à l'état pulvérulent.

10. Procédé selon la revendication 9, **caractérisé en ce que** le produit est un mélange de méthylolmélamines supérieures ayant une teneur en formaldéhyde libre inférieure à 0,8 % en poids et une teneur en eau inférieure à 5 % en poids.

11. Procédé de production de résines de mélamine fortement éthérifiée, **caractérisé en ce que** l'hexaméthylolmélamine produite au moyen d'un procédé selon l'une quelconque des revendications 1 à 8 est éthérifiée en continu ou de façon discontinue avec des alcools en C₁ à C₅ selon un rapport d'hexaméthylolmélamine:alcool en C₁ à C₅ de 1:8 à 1:30 dans des conditions maîtrisées de température et de pH, cela étant suivi d'une neutralisation et d'une élimination des composants volatils par distillation sous vide.

12. Procédé de production de résines de mélamine fortement éthérifiée, **caractérisé en ce qu'**on le met en oeuvre en continu d'une manière telle qu'un procédé en continu selon l'une quelconque des revendications 1 à 8 est combine avec une éthérification en continu d'hexaméthylolmélamine obtenue au moyen d'un procédé selon l'une quelconque des revendications 1 à 8 avec des alcools en C₁ à C₅ selon un rapport d'hexaméthylolmélamine:alcool en C₁ à C₅ de 1:8 à 1:30 dans des conditions maîtrisées de température et de pH, cela étant suivi d'une neutralisation et d'une élimination des composants volatils par distillation sous vide.

13. Procédé selon la revendication 11 ou 12, **caractérisé en ce que** l'alcool en C₁ à C₅ est du méthanol.

14. Procédé selon l'une quelconque des revendications 11 à 13, **caractérisé en ce que** le produit du procédé est de l'hexaméthoxyméthylolmélamine, de l'hexabutoxyméthylolmélamine ou des éthers mélangés d'alcools en C₁ à C₅ à base d'hexaméthylolmélamine, de préférence l'hexaméthoxyméthylolmélamine.

15. Procédé selon l'une quelconque des revendications 11 à 14, **caractérisé en ce que** le produit est de l'hexaméthoxyméthylolmélamine ayant une teneur en formaldéhyde libre inférieure à 0,08 % en poids.
